(19) Europäisches Patentamt
European Patent Office
Office européen des brevets

(11) Publication number: 0 238 243 A1

(12) # EUROPEAN PATENT APPLICATION

(21) Application number: 87302016.8

(22) Date of filing: 10.03.87

(51) Int.Cl.³: C 12 Q 1/04
C 12 Q 1/00, C 12 Q 1/12

(30) Priority: 12.03.86 GB 8606032

(43) Date of publication of application:
23.09.87 Bulletin 87/39

(84) Designated Contracting States:
AT BE CH DE ES FR GR IT LI LU NL SE

(71) Applicant: Cogent Limited
Temple Court 11 Queen Victoria Street
London, EC4N 4TP(GB)

(72) Inventor: James, Arthur
12 Wolseley Gardens
Newcastle upon Tyne NE2 1HR(GB)

(72) Inventor: Yeoman, Peter
7 Orchard Close
Rowlands GillTyne and Wear,NE39 1EQ(GB)

(74) Representative: Armitage, Ian Michael et al,
MEWBURN ELLIS & CO. 2/3 Cursitor Street
London EC4A 1BQ(GB)

(54) **Method for rapid testing of bacteria for identification.**

(57) A chromogenic substrate, such as an 8-hydroxyquinoline or 8-amino quinoline derivative, is enzymically cleaved by an enzyme of the microorganism to be identified. A chromogenic moiety is released, such as 8-hydroxyquinoline or 8-amino quinoline, and forms an insoluble coloured complex with metal ions present. When microorganisms are cultured on plates of nutrient material in the presence of such a chromogenic substrate and chelatable metal ions and the coloured complex is deposited intra or pericellularly and discrete areas of colour are produced.

EP 0 238 243 A1

0238243

METHOD FOR RAPID TESTING OF BACTERIA FOR IDENTIFICATION

The present invention relates to methods for rapid testing and identification of bacteria.

Detection of bacteria, and specific detection of *Escherichia coli* has both environmental and clinical applications, e.g. monitoring of water supplies, and clinical applications e.g. testing of faecal and urine samples. Many detection methods exploit enzymic reactions by either directly or indirectly measuring disappearace of a substrate or formation of a product.

Some rapid tests for monitoring drinking water are based on total coliform detection and have traditionally exploited common biochemical responses such as lactose fermentation, though more recently molecular hydrogen production has also been tested.

Methods with clinical applications have also been developed; in particular, the enzyme glutamic acid decarboxylase has been used for detection and measurement of bacteria and is reported to give a good measure of faecal coliform concentration. Detection and measurement of individual species of bacteria, such as *E. coli* or *Streptococcus faecalis*, have been recommended as more specific indicators of faecal contamination.

The processing of large numbers of urine samples constitutes a major part of the workload of most routine clinical microbiology laboratories. *E. coli* is the organism predominantly isolated from such samples, and it has been reported in over 80% of cases of significant bacteriuria (Mond et al, 1965, Lancet, i, 514-516; Trepeta and Edberg, 1984, J.Clin.Microbiol., 19, No.2, 172-174).

There is a need for a simple, rapid and reliable screening and identification test for bacteria, in particular *E. coli*, in urine and other samples. Various enzymic substrates have been examined and in particular chromogenic substances.

The use of orthonitrophenyl-β-D-galactoside (ONPG) for the rapid detection of the enzyme β-galactosidase in bacteria was extended by Kilian and Bulow (1976, Acta.Pathol.Microbiol.Scand., Sect.B 84, 245-241; 1979, Acta.Pathol.Microbiol. Scand., Sect.B 87, 271-276) to other glycosidases, the most interesting of which was β-glucuronidase. β-Glucuronidase activity, was demonstrated in 97% of the tested *E. coli* strains. Further it was noted, that among the Enterobacteriaceae and Vibrionaceae strains tested, only certain species of *Shigella* also contained β-glucuronidase. The specificity of β-glucuronidase as a marker enzyme for *E. coli* was further confirmed by a study which involved 400 strains of lactose fermenters, in which study 94% of *E. coli* strains were

found to contain this enzyme (Hansen and Yourassowsky, J. Clin.Microbiol.1984, 20, No.6, 1177-1179). It was found that most of the *Shigella* strains which exhibited β-glucuronidase activity were *Sh. sonnei*. These strains are potential late lactose fermenters and are thus, theoretically, confusable with *E. coli* (Henrichsen, 1985, Mast Matters, 26, 9-12; Henrichsen, 1986, Medical Laboratory Sciences, 43, 2-8).

The most commonly used substrates for the demonstration of β-glucuronidase activity in bacteria are those derived from the nitrophenols. *p*-Nitrophenyl-β-D-glucuronide (4-nitrophenyl-β-D-glucopyranosiduronic acid) has been extensively utilised but the corresponding *o*-nitrophenyl derivative appears less popular. Other substrates, capable of being hydrolysed by bacterial β-glucuronidase, are also available for analytical purposes and have been used either for enzyme assay or bacterial detection. β-D-glucuronide derivatives of phenolphthalein, chlorophenol, phenol and naphthol have also been used.

In the 1960's, 8-hydroxyquinoline (8-HQ) derivatives were used for detecting enzyme in locust crop fluid. However, 8-HQ was found to be bacteriostatic. It is now known that 8-HQ and possible certain derivatives can inhibit the enzyme RNA polymerase. This has been demonstrated in *E. coli* (Biochem. J. 147, 401 (1975)). 8-HQ is a colourless compound which has a high capacity for metal ion chelation. The chelates

formed tend to be stable within the appropriate pH range and are highly coloured.

In general terms the present invention provides the identification of bacteria by the reaction of an enzyme in the bacterium with a chromogenic substrate compound, this compound comprising a chromogenic moiety and an auxiliary group which is enzymically cleavable therefrom. The cleaved chromogenic moiety is then chelatable with a suitable metal to form a practically insoluble coloured chelate which concentrates intracellularly or pericellularly. The chelate is therefore easily visible and distinguished from neighbouring cells which do not have that enzymic activity.

In a preferred form, the present invention provides a method for the rapid identification of bacteria which comprises the use of substrates of the general formula:

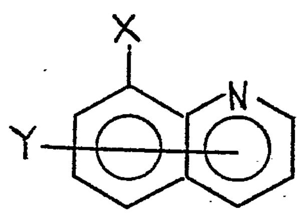

8-X-Q Formula I

in detecting a bacterial enzyme in bacteria as a visible concentration of a metal chelate in the cells, wherein X is a derivative of a hydroxyl or amino group providing a substrate moiety for an enzyme in the bacterium of interest, whereby the enzyme releases 8-hydroxyquinoline or 8-aminoquinoline which form a said chelate, Y being hydrogen or one

or more substituents in either ring which do not hinder either chelation or visualisation.

In a preferred form the method comprises:

a) multipointing a plurality of samples on a plate and incubating the samples in the presence of 8-XQ and a chelatable metal; and

b) inspecting the plate to identify a visible chelate concentration in the cells of bacteria containing enzymes for which 8-XQ is a substrate.

The derivative X is suitably chosen as a substrate for the specific enzyme of interest, whereby the enzymic action releases the metal chelating ligand. Suitably the metal to be chelated is of the first transition series or the alkaline earth metals and preferably it is selected from nickel, iron or cobalt. Suitably, chelation leads to deposition of a coloured complex intracellularly, or at most pericellularly.

Since 8-hydroxyquinolines may inhibit the enzyme RNA polymerase and hence prevent synthesis of messenger RNA coding for the enzyme to be detected it is preferable, where the substrate is an 8-hydroxyquinoline derivative, to use also a gratuitous inducer of the enzyme to be detected. For example the substrate moiety at X may be a sugar, such as glucoside, galactoside or a glucuronide. In these cases it is desirable to include with the chromogenic substrate compound an alkyl

β-thioglycoside to act as a gratuitous inducer of the glycosidase which is to be detected. Thus, where a β-galactosidase is to be detected by the method of the present invention iso-propyl thiogalactoside should be included to encourage the synthesis of β-galactosidase which might, otherwise, be inhibited by the presence of the 8-hydroxyquinoline derivative.

In order that the present invention may be better understood various features will be described by way of example only and with reference to the accompanying drawings, wherein:

Fig. 1 shows a plate containing 8-HQ-glucuronide;

Fig. 2a shows an aesculin plate;

Fig. 2b shows a plate containing 8-HQ-glucoside equivalent to Fig. 2a; and

Fig. 3 shows a close-up of a plate containing 8-HQ-sulphate.

<u>8-Hydroxyquinoline Identification Tests</u>

Agar plates containing peptone were prepared and equivalent amounts of 8-HQ-β-D-glucuronide (Sigma Chemical Co. Ltd.) and ferric ammonium citrate were incorporated into the plates.

Organisms were multipointed onto the agar plates and after being incubated overnight at 37°C the organisms

were rapidly identified. As shown in Fig. 1 glucuronidase-positive organisms were apparent by an intense black pigmentation located solely within the colonies and not spreading outside into the surrounding agar. Glucuronidase-negative organisms did not darken at all, and colonies of these are indicated by the stippled areas of Fig. 1.

The positive organisms were all identified as E. coli; this was confirmed by other tests, such as a full API20E testing.

Several hundred organisms were investigated using the 8-HQ-β-D-glucuronide technique, and the following results were obtained.

Between 80% and 85% of E. coli organisms were unequivocally detected by the plate method described above. These originate from urinary and other isolates.

No other enterobacteria gave a positive test result. More importantly, no Shigella species were detected, an advantage over the nitrophenyl-β-D-glucuronide method.

The 8-HQ-ferric ion complex appears to be deposited intracellular or, at least, pericellularly.

An important practical advantage resides in the ability to multipoint organisms on a single plate as the results of one colony do not interfere with the results of neighbouring colonies.

The plates are easily read with, for example, a 40-10 image analyser from Analytical Measuring Systems; the black and non-pigmented colonies giving good differentiation.

Other 8-HQ based substrates have been bought or synthesised. These have been tested in a similar fashion to the β-D-glucuronide substrate, the black pigmentation indicating colonies positive for the chosen enzyme.

Examples of derivatives include 8-HQ-β-D-glucoside testing for β-glucosidase, and 8-HQ sulphate for detecting sulphatase enzyme.

<u>8-HQ-β-D-Glucoside</u>

8-HQ-β-D-glucoside (Sigma Chemical Company Ltd.),together with ferric ammonium citrate, was incorporated into agar plates onto which the organisms were multi-pointed. The results obtained, as shown in Fig. 2b, paralleled conventional aesculin plates, as shown in Fig. 2a, in that the same organisms gave identical results for both tests.

However, the new technique is advantageous in that greater contrast between positive and negative colonies is obtained and the colour formed does not diffuse through the agar.

8-HQ-Sulphate

8-HQ-Sulphate was synthesised by adding technical chlorosulphonic acid (7.0g) dropwise, with constant stirring, to an ice-cooled solution of 8-HQ (7.0g) dissolved in pyridine (40 ml). The mixture was then made alkaline with concentrated aqueous potassium hydroxide (1:1). The solid precipitating was filtered off by suction and washed thoroughly with ether. The ester salt was extracted from the residue with hot alcohol (95%) and the alcoholic solution filtered through a heated funnel. The separated crystals were filtered with suction and washed with cold alcohol. Recrystallisation from a minimum of hot water yielded the pure ester potassium salt.

The sulphatase enzyme to which this test is directed is not common among Enterobacteriaceae. It is especially useful, however, in detecting Serratia marcescens and Providencia stuartii.

The test is performed as above and positive organisms identified by the black colonies formed, as shown in Fig.

3.

The present invention is not limited to the above examples. Other substrates have been, and are being, synthesised. One such is 8-HQ-ß-D-galactoside which has been used to identify galactosidase colonies as does the ONPG test. This is a valuable addition to existing multipoint techniques.

8-HQ-ß-D-galactoside is synthesised by the following method. Acetobromogalactose (3.0 g), 8-hydroxyquinoline (4.5 g), and potassium hydroxide (1.7 g) were dissolved in 50% of acetone-water and set aside for 24 h. The acetone was evaporated off and the residue, in benzene, was washed with M-sodium hydroxide, then water dried ($MgSO_4$), and evaporated. The 8-quinolyl tetra-O-acetylgalactoside was deacetylation with a catalytic amount of sodium methoxide in methanol to give 8-HQ-ß-D-galactoside. Recrystallisation from water to which a trace of dioxan had been added and drying in vacuo at 25° gave a monohydrate.

Use of 8-hydroxyquinoline-ß-D-galactoside alone, with chelating metal is largely unsuccessful for the detection of ß-galactosidase containing Enterobacteriaceae. Only very occasionally can the characteristic black colonies referred to previously be obtained. The vast majority of organisms known to be ß-galactosidase positive by ONPG-testing do not react in the testing procedure.

It is proposed that production of β-galactosidase, a known inducible enzyme in E. coli is repressed via 8-HQ mediated inhibition of RNA polymerase since the latter is known to be involved in synthesis of the messenger RNA coding for β-galactosidase and other enzymes of the lac operon.

When the testing procedure using 8-HQ-β-D-galactoside is repeated but with incorporation of a known gratuitous inducer of β-galactosidase, iso-propyl thiogalactoside, many black colonies are observed with known β-galactosidase positive organisms. No β-galactosidase negative colonies are visualised. Initial results indicate excellent agreement of classical (ONPG) and novel 8-HQ-β-D-galactoside methods.

Since other 8-HQ-derivatives may likewise fail to detect bacterial enzymes for similar reasons it may sometimes be desirable to include in the agar-based medium an alkyl-β-thioglycoside, or similar substance, corresponding to the glycosidase to be detected.

The invention is not limited to the use of 8-HQ. Initial studies indicate that 5-chloro-7-iodo-8-HQ (Aldrich Chemical Co.Ltd.), and 7-iodo-8-HQ-5-sulphonic acid (Sigma Chemicals) may be used as chelating moiety. After enzymic release this produces an intense blue colouration/precipitate which would be likely to appear as a dark blue-black deposit in cells.

Derivatives of 8-aminoquinoline (8-AQ) (Aldrich Chemicals), especially amino acyl-8-AQ's, would assist in identification of bacteria by demonstration of specific aminopeptidases.

The 8-AQ aminopeptidase substrates were synthesized by coupling the benzyloxycarbonyl (CBZ)-protected amino acid with 8-AQ by a mixed anhydride method. To a solution of the CBZ-protected amino acid (20 mmol) in 20.0 ml of anhydrous tetrahydrofuran (distilled from calcium hydride) and 350 µl of anhydrous triethylamine (distilled from calcium hydride) at 0°C, 300 µl of isobutylchloroformate was added dropwise over 5 min with stirring. A white precipate formed as stirring was continued for 20 min at 0°C. To this suspension, 8-AQ (144 mg) was added all at once. The resulting mixture was stirred at 0°C for 2 h and allowed to gradually warm to room temperature as stirring was continued overnight. The reaction mixture was then dissolved in ethyl acetate and washed three times with ice-cold 0.1 M HCl (100 ml), followed by one wash with saturated sodium bicarbonate (100 ml). The ethyl acetate layer was dried with saturated sodium chloride (100 ml) and then with anhydrous sodium sulfate. The ethyl acetate was removed in vacuo to yield the desired 8-(N-CBZ-aminoacylamido)-quinoline. The CBZ-protecting groups were removed by hydrogenolysis of 20 mmol of 8-(N-CBZ-aminoacylamido)-quinoline in 100 ml of solvent containing 250 mg of 10% palladium on carbon. The 10% palladium on carbon catalyst was removed by filtration, and the solvent

was removed in vacuo to yield the desired aminoacyl 8-aminoquinoline.

Other organic moieties, capable of being conjugated to enzymatically labile groups which, after enzymatic hydrolysis, in the presence of an appropriate metal give a virtually insoluble chelate, may be similarly employed for identification.

Selection of various chelating agents and their derived substrates for use in multipoint inoculation systems will build up enzymatic profile characteristics of particular microbial species and lead to their rapid identification.

CLAIMS

1. A method of identifying a microorganism in a sample, said method comprising contacting the sample with a chromogenic compound having a chromogenic moiety and an auxiliary group, said compound being a substrate for an enzyme in said microorganism so that the compound is enzymatically cleaved to release the chromogenic moiety from the auxiliary group whereby the microorganism is identified by visualisation of the released chromogenic moiety; characterised in that the chromogenic moiety is chelated with a chelatable metal ion to form a substantially insoluble coloured chelate which can be visualised intracellularly or pericellularly of the microorganism.

2. A method according to claim 1 wherein the chromogenic substrate has the formula

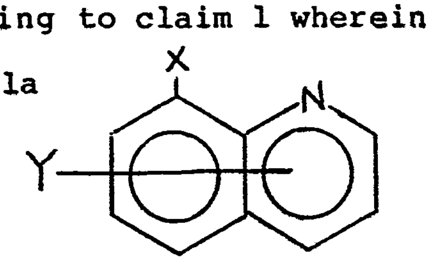

wherein X is a derivative of a hydroxyl or amino group providing an auxiliary group which is a substrate moiety for an enzyme in the microorganism to be identified, and Y is hydrogen or one or more substituents in either ring which do not hinder either chelation or visualisation, and wherein the chromogenic moiety cleaved is an 8-hydroxyquinoline, 8-aminoquinoline or derivative thereof.

3. A method according to claim 2 wherein X is a β-D-glucuronidyl, β-D-glucosyl, sulphate, β-D-galactosyl or amino acyl group, the auxiliary group so provided being a substrate for a glucuronidase, glucosidase, sulphatase, galactosidase or amino peptidase respectively in the microorganism to be detected.

4. A method according to claim 3, for the detection of Escherichia coli wherein X is β-D-glucuronidyl and the enzyme in the Escherichia coli for which the glucuronidyl group is a substrate is a glucuronidase.

5. A method according to any one of the preceding claims wherein the sample is contacted with a plate of nutrient medium incorporating the chromogenic substrate and chelatable metal ion, and the plate is incubated.

6. A method according to claim 5 wherein a plurality of said samples is multipointed on the plate.

7. A method according to any one of the preceding claims wherein the chelatable metal ion is of the first transition series or the alkaline earth metals.

8. A method according to claim 7 wherein the chelatable metal ion is of nickel, iron or cobalt.

9. A method according to any one of the preceding claims wherein the microorganism is contacted with an inducer of the enzyme in addition to the chromogenic substrate.

10. A method according to claim 9 wherein, the chromogenic substrate is a glycosyl derivative of 8-hydroxyquinoline and the inducer is an alkyl β-thioglycoside.

11. A culture medium for use in solid phase culture of a microorganism to be identified, said medium comprising a chromogenic substrate compound having a chromogenic moiety and an auxiliary group enzymatically cleavable therefrom by an enzyme of the microorganism to be identified, whereby the microorganism can be visualised by the release of the chromogenic moiety on incubation of the medium with the sample; characterised in that the medium further comprises a metal ion chelatable with said chromogenic moiety when cleaved from its auxiliary group, to form a coloured chelate which is substantially insoluble in the medium.

12. A culture medium according to claim 11 wherein the chromogenic substrate has the formula

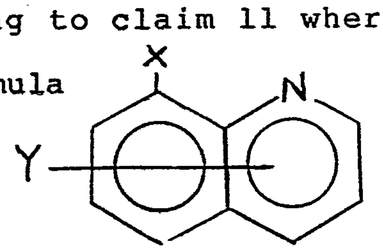

wherein X is a derivative of a hydroxyl or amino group providing an auxiliary group which is a substrate moiety for an enzyme in the microorganism to be identified, and Y is

hydrogen or one or more substituents in either ring which do not hinder either chelation or visualisation of the chelate in the microorganism.

13. A culture medium according to claim 12 wherein the chromogenic substrate is 8-hydroxyquinoline-β-D-glucuronide, 8-hydroxyquinoline-β-D-glucoside, 8-hydroxyquinoline sulphate, 8-hydroxyquinoline-β-D-galactoside or an 8-aminoquinoline derivative carrying an amino acid or peptide on the amino nitrogen group.

14. A culture medium according to any one of claims 11 to 13 wherein the chelatable metal ion is of the first transition series or the alkaline earth metals.

15. A culture medium according to claim 14 wherein the chelatable metal ion is of nickel, iron or cobalt.

16. A culture medium according to any one of claims 11 to 15 further comprising an inducer for the enzyme.

17. A culture medium according to claim 16 wherein the chromogenic substrate is a glycosyl derivative of 8-hydroxyquinoline and the inducer is an alkyl β-thioglycoside.

Fig.1.

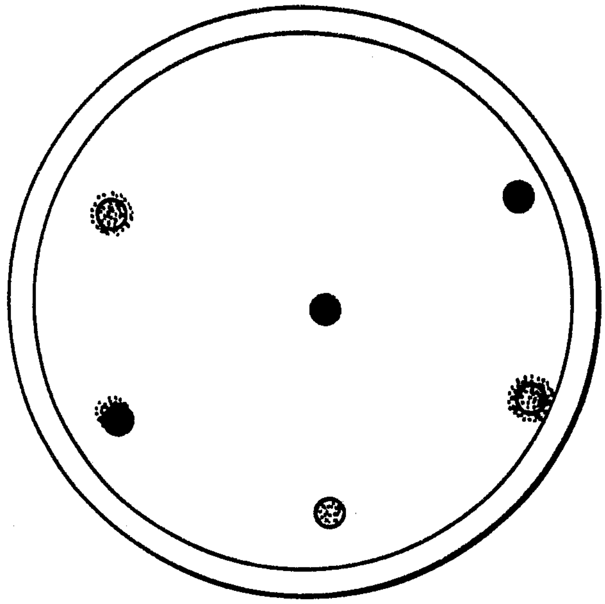

Fig.3.

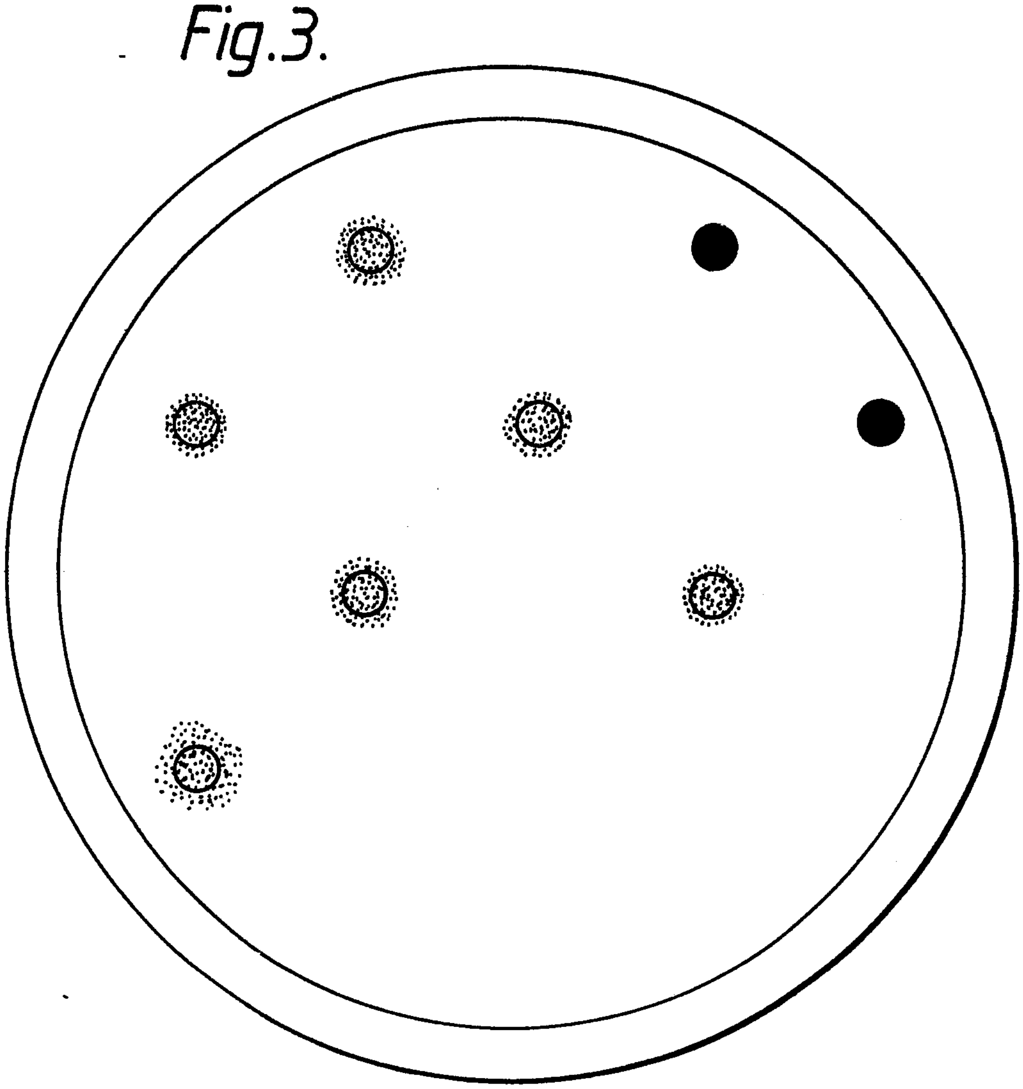

Fig. 2A.

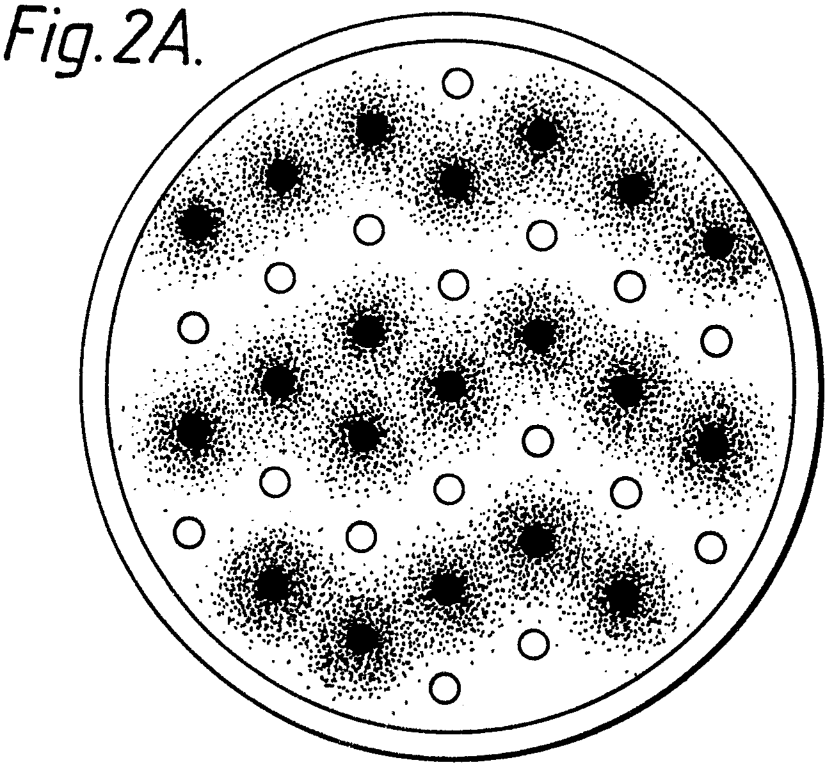

Fig. 2B.

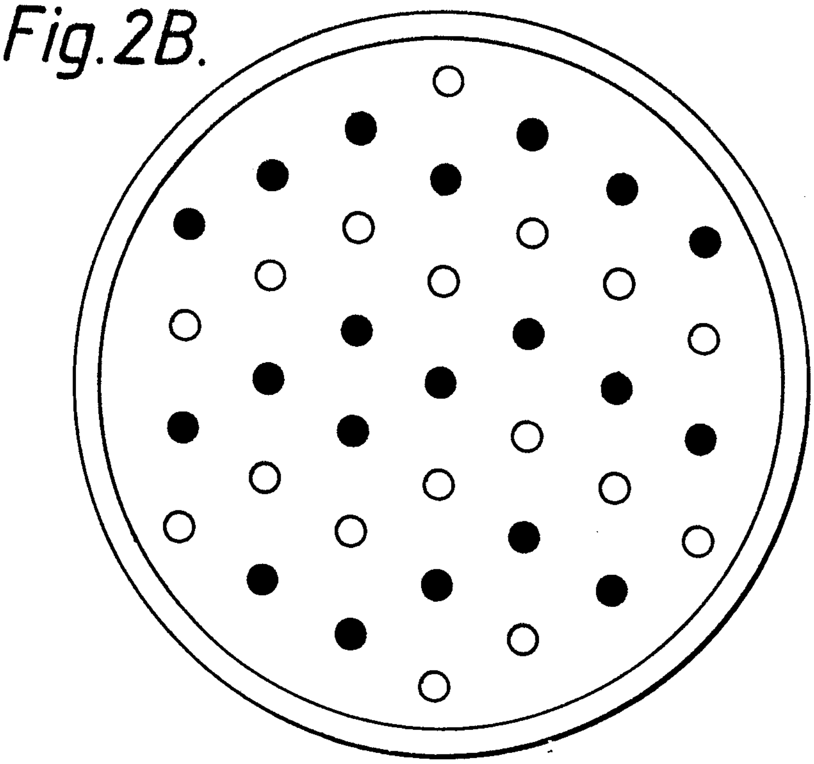

European Patent Office

# EUROPEAN SEARCH REPORT

0238243

Application number

EP 87 30 2016

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 025 467 (A. RAMBACH)<br>* Whole document *<br>--- | 1-17 | C 12 Q 1/04<br>C 12 Q 1/00<br>C 12 Q 1/12 |
| Y | CHEMICAL ABSTRACTS, vol. 42, no. 21, 10th November 1948, abstract no. 8913c, Columbus, Ohio, US; J. FRIEDENWALD et al.: "The histochemical localization of glucuronidase", & J. CELLULAR COMP. PHYSIOL. 31, 303-9 (1948)<br>* Abstract *<br>--- | 1-17 | |
| Y | GB-A-1 107 790 (BOEHRINGER & SÖHNE GmbH)<br>* Page 2, radical (d) and lines 21-23; page 4, lines 16-20 *<br>--- | 1-17 | |
| Y | WO-A-8 505 378 (FLUORODIAGNOSTIC LTD PARTNERSHIP)<br>* Whole document *<br>--- | 1-17 | TECHNICAL FIELDS SEARCHED (Int. Cl.4)<br>C 12 Q |
| A | GB-A-2 031 949 (AMERICAN HOME PRODUCTS CORP.)<br>--- | | |
| A | CHEMICAL ABSTRACTS, vol. 76, no. 3, 17th January 1972, page 429, abstract no. 10025v, Columbus, Ohio, US; A.Kh. BATALIN et al.: "Use of 8-hydroxyquinoline derivatives in chemical analysis", & UCH. ZAP. OMSK. GOS. PEDAGOG. INST. 1970, no. 29, 171-7<br>* Abstract *<br>--- | 1,2 | |

The present search report has been drawn up for all claims

| Place of search | Date of completion of the search | Examiner |
|---|---|---|
| THE HAGUE | 18-06-1987 | OSBORNE H.H. |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document